# EUROPEAN PATENT APPLICATION

(11) **EP 1 219 617 A1**
(43) Date of publication of application: **03.07.2002**
(21) Application number: 00961098.1
(22) Date of filing: 20.09.2000
(51) Int. Cl.: C07D 323/00, C07D 319/12

(54) **PROCESS FOR THE PREPARATION OF CYCLIC LACTIC ACID OLIGOMERS**

(30) Priority: 20.09.1999 JP 26573299
(71) Applicant: AMATO PHARMACEUTICAL PRODUCTS, LTD., Fukuchiyama-shi, Kyoto 620-0932 (JP)
(72) Inventor: WATANABE, Mikio, Hadano-shi, Kanagawa 257-0002 (JP); TAKANO, Jiro, Hadano-shi, Kanagawa 259-1322 (JP); ISHIHARA, Yoshimi, Atsugi-shi, Kanagawa 243-0035 (JP); MURAKAMI, Masahiro, Osaka 547-0026 (JP)
(74) Representative: Sternagel, Fleischer, Godemeyer & Partner Patentanwälte
(86) International application number: PCT/JP00/06399
(87) International publication number: WO 01/21613

(57) **Abstract**

The object of the present invention is to provide a novel method for producing cyclic lactic acid oligomers at a high yield, and to provide a cyclic lactic acid oligomer produced by said method. According to the present invention, there is provided a method for producing a cyclic lactic acid oligomer, which comprises:
(i) a first heating process for dehydration condensation of lactic acids by heating, which comprises dehydration condensation of lactic acids under conditions of a pressure and a temperature which allow by-product water to be removed by distillation while avoiding distillation of lactides; and
(ii) a second heating process for generating a dehydrated condensate of lactic acid, which comprises heating the reaction product from said first heating process to a temperature higher than that of the first heating process, reducing the pressure to 100mmHg or lower under conditions of a pressure and a temperature which allow by-product water to be removed by distillation while avoiding distillation of lactides, and further continuing the reaction by heating under the reduced pressure; as well as a cyclic lactic acid oligomer produced by said production method.

## Description

### TECHNICAL FIELD

The present invention relates to a method for producing a cyclic lactic acid oligomer, and a cyclic lactic acid oligomer produced by the production method.

### BACKGROUND ART

There is known a method for producing a lactic acid oligomer by dehydration condensation reaction of lactic acids under reduced pressure. Lactic acid oligomers obtained by this method comprise both chain oligomers and cyclic oligomers.

As a method for obtaining cyclic oligomers, there is known a method which comprises, during dehydration condensation reaction of lactic acids, heating at 145°C for 3 hours under normal pressure, reducing pressure to 150mmHg, heating for 3 hours under the reduced pressure, and then heating at 185°C for 1.5 hours under a pressure of 3mmHg (Japanese Patent Application Laying-Open (kokai) No. 9-227383).

In this method, however, the generation yield of cyclic oligomers is low, and there still remains some room for improvement of the yield.

### DISCLOSURE OF THE INVENTION

The object of the present invention is to provide a novel method for producing cyclic lactic acid oligomers at a high yield, and to provide a cyclic lactic acid oligomer produced by said method.

As a result of focused research to achieve the aforementioned object, the present inventors have found that a cyclic lactic acid oligomer can be produced at a high yield by dehydration condensation reaction of lactic acids under conditions of a certain pressure and a certain temperature which allow by-product water to be removed by distillation while avoiding distillation of lactides, thereby providing the present invention.

Thus, according to the present invention, there is provided a method for producing a cyclic lactic acid oligomer, which comprises:
(i) a first heating process for dehydration condensation of lactic acids by heating, which comprises dehydration condensation of lactic acids under conditions of a pressure and a temperature which allow by-product water to be removed by distillation while avoiding distillation of lactides; and
(ii) a second heating process for generating a dehydrated condensate of lactic acid, which comprises heating the reaction product from said first heating process to a temperature higher than that of the first heating process, reducing the pressure to 100mmHg or lower under conditions of a pressure and a temperature which allow by-product water to be removed by distillation while avoiding distillation of lactides, and further continuing the reaction by heating under the reduced pressure.
   Preferably, in the first heating process (i), lactic acids are subjected to dehydration condensation reaction by heating to a temperature of 150°C or lower under a pressure of 10 to 760mmHg. More preferably, in the first heating process (i), lactic acids are subjected to dehydration condensation reaction by heating to a temperature ranging from 120°C to 140°C under a pressure of 350 to 400mmHg.
   Preferably, in the second heating process (ii), the reaction product from the first heating process is heated to 145°C or higher, the reaction pressure is reduced to 100mmHg or lower at a depressurization rate of 0.5 to 1mmHg/min, and the reaction is further continued under the reduced pressure and at a temperature of 145°C or higher so as to generate a dehydrated condensate of lactic acid. More preferably, in the second heating process (ii), the reaction product from the first heating process is heated to 150°C to 160°C, and while the reaction pressure is reduced to 15 to 20mmHg at a depressurization rate of 0.5 to 1mmHg/min, by-product water is removed by distillation while avoiding distillation of lactides, and after the reaction pressure is reduced to 15 to 20mmHg, the reaction is further continued under the same pressure and at a reaction temperature of 150°C to 160°C so as to generate a dehydrated condensate of lactic acid
   Preferably, the method for producing a cyclic lactic acid oligomer according to the present invention further comprises:
(iii) a third heating process for generating a cyclic lactic acid oligomer, which comprises cyclization of a chain lactic acid oligomer in the reaction product from said second heating process by heating under a pressure lower than that of said second heating process.

Preferably, in the third heating process (iii), the reaction product from the second heating process is heated at 150°C to 160°C under a pressure of 0.1 to 5mmHg.

According to the particularly preferred embodiment of the present invention, there is provided a method for producing a cyclic lactic acid oligomer, which comprises:
(i) a first heating process, which comprises heating lactic acids to a temperature ranging from 120°C to 140°C under a pressure of 350 to 400mmHg for dehydration condensation reaction, while removing by-product water by distillation, and avoiding distillation of lactides;
(ii) a second heating process, which comprises heating the reaction product from said first heating process to a temperature of 150°C to 160°C, reducing the reaction pressure to 15 to 20mmHg at a depressurization rate of 0.5 to 1mmHg/min, removing by-product water by distillation while avoiding distillation of lactides, and after the reaction pressure is reduced to 15 to 20mmHg, further continuing the reaction under the same pressure and at a reaction temperature of 150°C to 160°C so as to generate a dehydrated condensate of lactic acid; and
(iii) a third heating process, which comprises cyclizing a chain lactic acid oligomer in the reaction product from said second heating process by heating at 150°C to 160°C under a pressure of 0.1 to 5mmHg so as to generate a cyclic oligomer.

In a preferred embodiment of the present invention, cyclic lactic acid oligomers are selectively produced while substantially no chain lactic acid oligomers are produced. Preferably in this case, the ratio of cyclic lactic acid oligomers to total lactic acid oligomers in the reaction product is 80% by weight or more.

According to another aspect of the present invention, there is provided a cyclic lactic acid oligomer produced by the method for producing a cyclic lactic acid oligomer of the present invention. Preferably there is provided a cyclic lactic acid oligomer which is substantially free of chain lactic acid oligomers.

### BRIEF DESCRIPTION OF THE DRAWINGS

Figure 1 shows an MS spectrum of the reaction product obtained by Example 1.
Figure 2 shows a general view of NMR of the reaction product obtained by Example 1.
Figure 3 shows a partial scale view of Figure 2.
Figure 4 shows a partial scale view of Figure 2.

### THE BEST MODE FOR CARRYING OUT THE INVENTION

Embodiments and methods for carrying out the present invention are described in detail below.

The method for producing a cyclic lactic acid oligomer of the present invention comprises:
(i) a first heating process for dehydration condensation of lactic acids by heating, which comprises dehydration condensation of lactic acids under conditions of a pressure and a temperature which allow by-product water to be removed by distillation while avoiding distillation of lactides; and
(ii) a second heating process for generating a dehydrated condensate of lactic acid, which comprises heating the reaction product from said first heating process to a temperature higher than that of the first heating process, reducing the pressure to 100mmHg or lower under conditions of a pressure and a temperature which allow by-product water to be removed by distillation while avoiding distillation of lactides, and further continuing the reaction by heating under the reduced pressure.

The lactic acid used as a raw material in the present invention may be any one of D-lactic acid, L-lactic acid and DL-lactic acid, and these lactic acids may also be used alone or in combination of two or more.

The first heating process in the present invention is a process of dehydration condensation of lactic acids by heating under conditions of a pressure and a temperature which allow by-product water to be removed by distillation while avoiding distillation of lactides. In the reaction of the first heating process, by-product water generated by dehydration condensation of lactic acids is removed by distillation in order to achieve smooth progression of the reaction, and in this case, a pressure and a temperature are determined so as to avoid distillation of lactide that is a dehydrated condensate of 2 molecules of lactic acid.

The reaction pressure may be normal pressure or reduced pressure, and reduced pressure is preferable. The reaction pressure is specifically 10 to 760mmHg, preferably 300 to 500mmHg, and more preferably 350 to 400mmHg. The reaction temperature depends on pressure conditions, but generally is within a range of 100°C to 150°C, preferably 120°C to 140°C.

The reaction period of the first heating process is not particularly limited, but is generally 3 to 12 hours, preferably 5 to 6 hours.

A reaction product containing, as a main component, a dehydrated condensate of 3 to 23 molecules of lactic acid, is generated from the reaction of the first heating process.

According to the method of the present invention, after completion of the first heating process mentioned above, in the second heating process, the reaction product is heated to a temperature higher than the reaction temperature of the first heating process, e.g. 145°C or higher, preferably 150°C to 180°C, more preferably 150°C to 160°C so as to obtain oligomers having a higher average polymerization degree, and at the same time the reaction pressure is reduced to 100mmHg or lower, preferably 10 to 80mmHg, more preferably 15 to 20mmHg, and then dehydration condensation reaction is further continued.

As in the case of the reaction of the first heating process, the reaction of the second heating process is carried out under conditions such that allow by-product water to be removed by distillation, but distillation of lactides is avoided, so as to smoothly progress the reaction.

From the studies of the present inventors, it was found that a rate of reducing reaction pressure (depressurization rate) within the above-stated range (i.e. 100mmHg or lower) should be maintained at no higher than 5mmHg/min in order to avoid distillation of lactides and to increase reaction efficiency. The depressurization rate is preferably in a range from no lower than 0.25mmHg/min to no higher than 5mmHg/min, more preferably from no lower than 0.25mmHg/min to no higher than 4mmHg/min, still more preferably from no lower than 0.5mmHg/min to no higher than 3mmHg/min, and particularly preferably from no lower than 0.5mmHg/min to no higher than 1mmHg/min. A depressurization rate lower than the range stated above is not preferable since time required for reducing the pressure to a designated pressure is longer, and a depressurization rate of 5mmHg/min or higher is not preferable since lactides are removed by distillation with by-product water.

After the reaction pressure is reduced to 100mmHg or lower, the reaction is further continued at this reaction pressure. The reaction period of this case is 3 to 12 hours, preferably 5 to 6 hours.

There is obtained from the reaction of the second heating process, a lactic acid oligomer having an average polymerization degree of 3 to 30, preferably 3 to 23. The ratio of cyclic lactic acid oligomers to total lactic acid oligomers in the reaction product from the second heating process is generally 70% by weight or more, and for example, around 70% to 80% by weight.

In a preferred embodiment of the present invention, a third heating process is carried out after completion of the aforementioned second heating process. The third heating process is a process where a chain lactic acid oligomer in the reaction product from the second heating process is cyclized by heating at a pressure still lower than that of the second heating process so as to generate a cyclic lactic acid oligomer.

The reaction pressure of the third heating process is preferably 0.1 to 5mmHg, more preferably 0.25 to 5mmHg, still more preferably 0.5 to 3mmHg, and particularly preferably 0.5 to 1mmHg. The reaction temperature of the third heating process is preferably 145°C to 180°C, and more preferably 150°C to 160°C.

The reaction is continuously performed under such pressure and temperature conditions. The reaction period is 3 to 12 hours, preferably 5 to 6 hours. By-product water generated in this case is also removed by distillation. In this case, it is preferable to avoid distillation of lactides, but it is not necessary to set the depressurization rate especially low since almost no lactides are contained in the reaction product.

The reaction of the third heating process generates a cyclic lactic acid oligomer having an average polymerization degree of 3 to 30, preferably 3 to 23. The ratio of cyclic lactic acid oligomers to total lactic acid oligomers in the reaction product from the third heating process is generally 90% by weight or more, and preferably 99% by weight or more.

The cyclic lactic acid oligomer produced by the method of the present invention is assumed to have the following chemical structure: 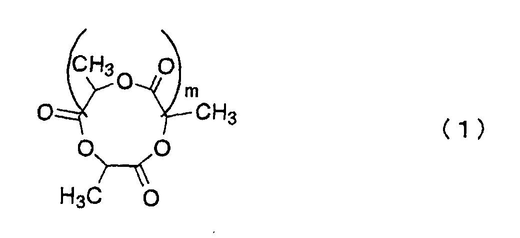 wherein m is an integer of 1 to 28, and preferably m is an integer of 1 to 19.

In a preferred embodiment of the method for producing a cyclic lactic acid oligomer of the present invention, cyclic lactic acid oligomers can selectively be produced while substantially no chain lactic acid oligomers are produced. The term "substantially no chain lactic acid oligomers are produced" is used herein to mean that the ratio of cyclic lactic acid oligomers to total lactic acid oligomers in a reaction product is 80% by weight or more, preferably 90% by weight or more, more preferably 95% by weight or more, and particularly preferably 99% by weight or more.

The present invention also relates to a cyclic lactic acid oligomer produced by the aforementioned method for producing a cyclic lactic acid of the present invention. In a preferred embodiment of the present invention, a mixture of cyclic lactic acid oligomers substantially free of chain lactic acid oligomers can be produced. The term "a mixture of cyclic lactic acid oligomers substantially free of chain lactic acid oligomers" is used herein to mean that the ratio of cyclic lactic acid oligomers to total lactic acid oligomers in the mixture is 80% by weight or more, preferably 90% by weight or more, more preferably 95% by weight or more, and particularly preferably 99% by weight or more.

The mixture of cyclic lactic acid oligomers produced by the method of the present invention (or a single substance obtained by purification from the mixture) is useful as a tumor cell growth inhibiting agent, an antineoplastic agent, a preventive agent against cancer metastasis, a QOL improving agent for cancer patients, an immune activating agent, and the like, and the mixture can also be used for prevention and/or treatment of diabetes or diabetes complications since it has an action of reducing blood sugar level. Moreover, the mixture of cyclic lactic acid oligomers produced by the method of the present invention (or a single substance obtained by purification from the mixture) has an action of repressing excessive appetite and promoting basal metabolism, and so it can be used also as a medicament useful for improvement and/or prevention of adiposis and enhancement of effects of kinesitherapy, and is also useful as an agent for promoting glycogen accumulation or an agent for enhancing physical fitness. Furthermore, a cyclic lactic acid oligomer produced by the method of the present invention is useful not only as a medicament, but also as health foods or diet supplements including beverages, which is generally called soft drinks, drinkable preparations, health foods, specific hygienic foods, functional foods, function activating foods, nutritional supplementary foods, supplements, feed, feed additives, and the like.

The present invention is further described in the following examples. It is apparent to those skilled in the art that materials, usage, proportion, treatment, treatment process and the like shown in the following examples can be modified as appropriate, as long as the modifications are within the spirit and scope of the invention, and the examples are not intended to limit the scope of the invention.

### EXAMPLES

### Example 1.

10.0g of (s)-(+)-lactic acid was placed in a 100ml (internal volume) eggplant-shaped flask, which was then set on a rotary evaporator. The pressure in the flask was controlled to be 350 to 400mmHg, and the flask was heated to 140°C, followed by reaction at the same pressure and the same temperature for 6 hours (the first heating process). By-product water generated from this reaction was removed by distillation. Almost no lactides were removed by distillation outside the system under the above reaction conditions.

Then, the reaction temperature was raised to 150 to 160°C, and the reaction pressure was gradually reduced from 400mmHg to 15 to 20mmHg over about 6 hours (depressurization rate: 1mmHg/min). At this depressurization rate, by-product water was removed by distillation, but almost no lactides were removed by distillation. After that, the pressure was maintained at 15 to 20mmHg and the reaction was continuously performed for 6 hours (the second heating process).

Subsequently, the pressure was reduced to 1 to 3mmHg over 30 minutes, and then the reaction was continuously performed at a reaction temperature of 160°C for 5 hours (the third heating process).

After completion of the reaction, the reaction product was analyzed and as a result, there was obtained 6.80g (yield 85%) of cyclic oligomer having an average polymerization degree of 3 to 21.

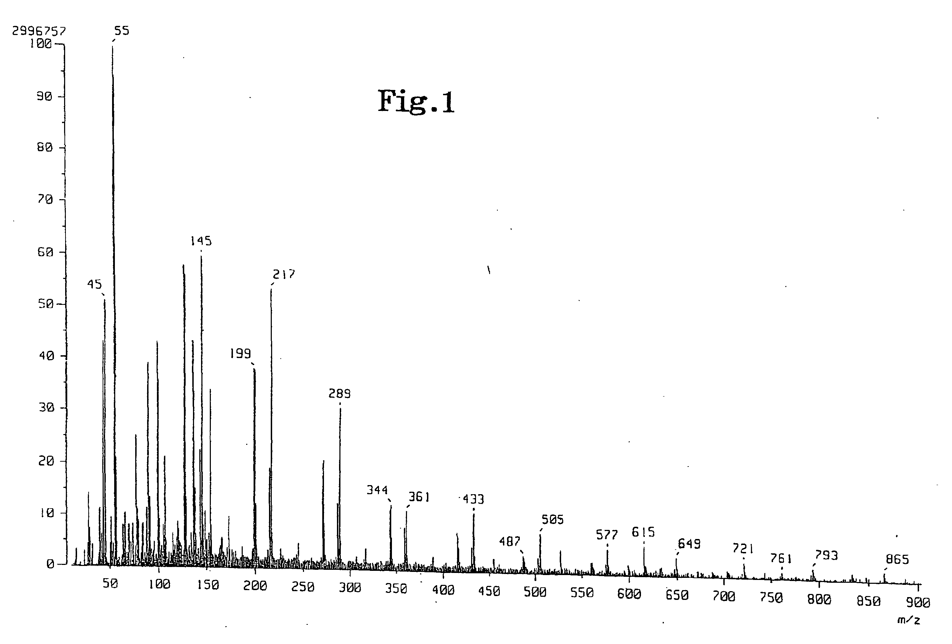

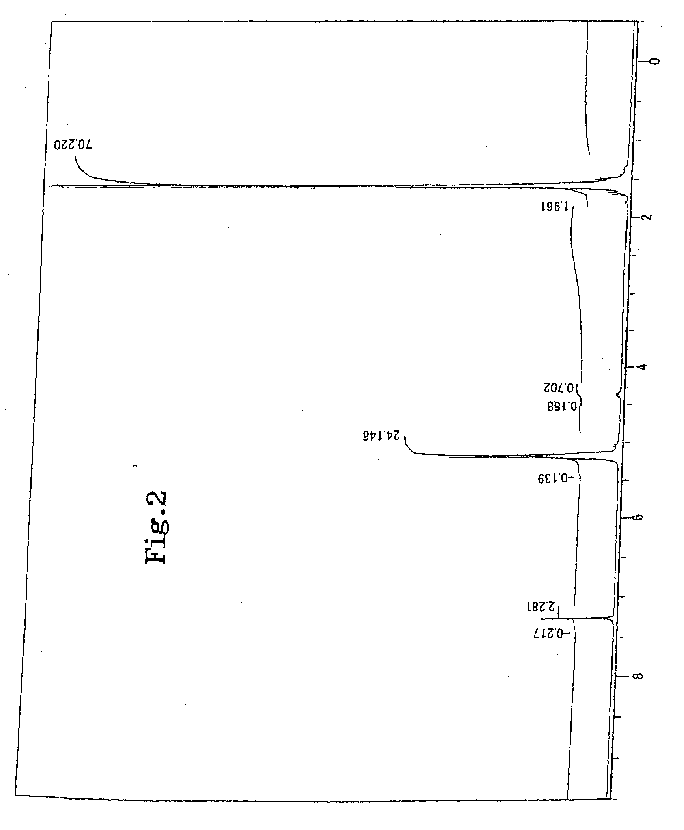

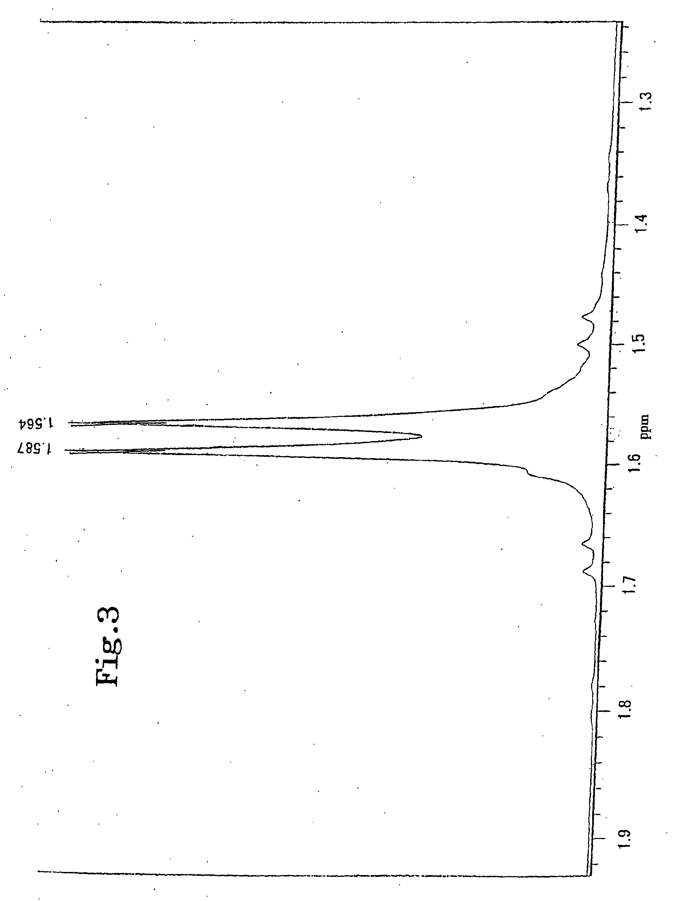

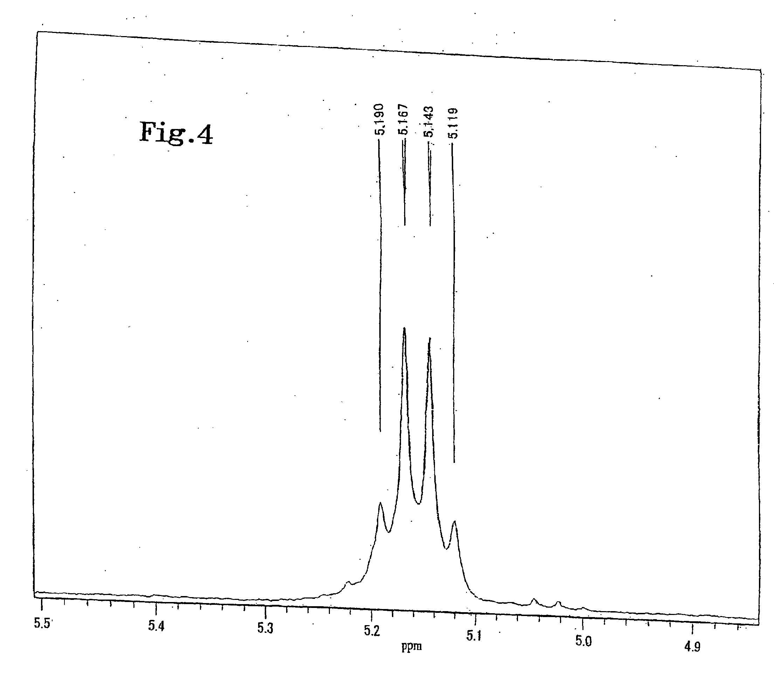

An MS spectrum of the reaction product obtained in Example 1 is shown in Figure 1. A general view of NMR of the reaction product obtained in Example 1 is shown in Figure 2, and partial scale views of Figure 2 are shown in Figures 3 and 4.

### Example 2.

10.0g of (s)-(+)-lactic acid was placed in a 100ml (internal volume) eggplant-shaped flask, which was then set on a rotary evaporator. The pressure in the flask was controlled to be 760mmHg, and the flask was heated to 140°C, followed by reaction at the same pressure and the same temperature for 10 hours (the first heating process). By-product water generated from this reaction was removed by distillation. Almost no lactides were removed by distillation outside the system under the above reaction conditions.

Then, the reaction temperature was raised to 150 to 160°C, and the reaction pressure was gradually reduced from 760mmHg to 15 to 20mmHg over about 12 hours (depressurization rate: 1mmHg/min). At this depressurization rate, by-product water was removed by distillation, but almost no lactides were removed by distillation. After that, the pressure was maintained at 15 to 20mmHg and the reaction was continuously performed for 6 hours (the second heating process).

Subsequently, the pressure was reduced to 1 to 3mmHg over 30 minutes, and then the reaction was continuously performed at a reaction temperature of 160°C for 5 hours (the third heating process).

After completion of the reaction, the reaction product was analyzed and as a result, there was obtained 6.80g (yield 85%) of cyclic oligomer having an average polymerization degree of 3 to 21.

An MS spectrum and NMR of the reaction product obtained in Example 2 were the same as those of the reaction product obtained in Example 1.

### Example 3.

10.0g of (s)-(+)-lactic acid was placed in a 100ml (internal volume) eggplant-shaped flask, which was then set on a rotary evaporator. The pressure in the flask was controlled to be 350 to 400mmHg, and the flask was heated to 140°C, followed by reaction at the same pressure and the same temperature for 6 hours (the first heating process). By-product water generated from this reaction was removed by distillation. Almost no lactides were removed by distillation outside the system under the above reaction conditions.

Then, the reaction temperature was raised to 150 to 160°C, and the reaction pressure was gradually reduced trom 400mmHg to 70mmHg over about 5.5 hours (depressurization rate: 1mmHg/min). At this depressurization rate, by-product water was removed by distillation, but almost no lactides were removed by distillation. After that, the pressure was maintained at 70mmHg and the reaction was continuously performed for 10 hours (the second heating process).

Subsequently, the pressure was reduced to 1 to 3mmHg over 70 minutes, and then the reaction was continuously performed at a reaction temperature of 160°C for 5 hours (the third heating process).

After completion of the reaction, the reaction product was analyzed and as a result, there was obtained 6.80g (yield 85%) of cyclic oligomer having an average polymerization degree of 3 to 21.

An MS spectrum and NMR of the reaction product obtained in Example 3 were the same as those of the reaction product obtained in Example 1.

### Comparison Example 1.

Comparison Example 1 was carried out in the same manner as in Example 1 with the exception that depressurization rate in the second heating process was set to be 5mmHg/min. In the depressurization operation, lactides were removed by distillation with by-product water, and as a result, the yield of cyclic oligomer was decreased to 60%.

### INDUSTRIAL APPLICABILITY

According to the present invention, a cyclic lactic acid oligomer having an average polymerization degree of 3 to 30, preferably 3 to 21, can be produced at a high yield from lactic acids without using a catalyst. Moreover, a cyclic lactic acid oligomer produced by the production method of the present invention is useful as a tumor cell growth inhibiting agent, antineoplastic agent, preventive agent against cancer metastasis, QOL improving agent for cancer patients, immune activating agent, therapeutic agent for diabetes, antiobestic agent, a agent for promoting glycogen accumulation or an agent for enhancing physical fitness. Furthermore, the cyclic lactic acid oligomer is useful not only as a medicament, but also as various types of health foods and diet supplements including soft drinks, drinkable preparations, health foods, specific hygienic foods, functional foods, function activating foods, nutritional supplementary foods, supplements, feed, feed additives, and the like.

## Claims

1. A method for producing a cyclic lactic acid oligomer, which comprises:
(i) a first heating process for dehydration condensation of lactic acids by heating, which comprises dehydration condensation of lactic acids under conditions of a pressure and a temperature which allow by-product water to be removed by distillation while avoiding distillation of lactides; and
(ii) a second heating process for generating a dehydrated condensate of lactic acid, which comprises heating the reaction product from said first heating process to a temperature higher than that of the first heating process, reducing the pressure to 100mmHg or lower under conditions of a pressure and a temperature which allow by-product water to be removed by distillation while avoiding distillation of lactides, and further continuing the reaction by heating under the reduced pressure.

2. The method for producing a cyclic lactic acid oligomer according to claim 1, wherein in the first heating process (i), lactic acids are subjected to dehydration condensation reaction by heating to a temperature of 150°C or lower under a pressure of 10 to 760mmHg.

3. The method for producing a cyclic lactic acid oligomer according to claim 1 or 2, wherein in the first heating process (i), lactic acids are subjected to dehydration condensation reaction by heating to a temperature ranging from 120°C to 140°C under a pressure of 350 to 400mmHg.

4. The method for producing a cyclic lactic acid oligomer according to any one of claims 1 to 3, wherein in the second heating process (ii), the reaction product from the first heating process is heated to 145°C or higher, the reaction pressure is reduced to 100mmHg or lower at a depressurization rate of 0.5 to 1mmHg/min, and the reaction is further continued under the reduced pressure and at a temperature of 145°C or higher so as to generate a dehydrated condensate of lactic acid.

5. The method for producing a cyclic lactic acid oligomer according to any one of claims 1 to 4, wherein in the second heating process (ii), the reaction product from the first heating process is heated to 150°C to 160°C, and while the reaction pressure is reduced to 15 to 20mmHg at a depressurization rate of 0.5 to 1mmHg/min, by-product water is removed by distillation while avoiding distillation of lactides, and after the reaction pressure is reduced to 15 to 20mmHg, the reaction is further continued under the same pressure and at a reaction temperature of 150°C to 160°C so as to generate a dehydrated condensate of lactic acid.

6. The method for producing a cyclic lactic acid oligomer according to any one of claims 1 to 5, which further comprises:
(iii) a third heating process for generating a cyclic lactic acid oligomer, which comprises cyclization of a chain lactic acid oligomer in the reaction product from said second heating process by heating under a pressure lower than that of said second heating process.

7. The method for producing a cyclic lactic acid oligomer according to claim 6, wherein in the third heating process (iii), the reaction product from the second heating process is heated at 150°C to 160°C under a pressure of 0.1 to 5mmHg.

8. The method for producing a cyclic lactic acid oligomer according to claim 1, which comprises:
(i) a first heating process, which comprises heating lactic acids to a temperature ranging from 120°C to 140°C under a pressure of 350 to 400mmHg for dehydration condensation reaction, while removing by-product water by distillation, and avoiding distillation of lactides;
(ii) a second heating process, which comprises heating the reaction product from said first heating process to a temperature of 150°C to 160°C, reducing the reaction pressure to 15 to 20mmHg at a depressurization rate of 0.5 to 1mmHg/min, removing by-product water by distillation while avoiding distillation of lactides, and after the reaction pressure is reduced to 15 to 20mmHg, further continuing the reaction under the same pressure and at a reaction temperature of 150°C to 160°C so as to generate a dehydrated condensate of lactic acid; and
(iii) a third heating process, which comprises cyclizing a chain lactic acid oligomer in the reaction product from said second heating process by heating at 150°C to 160°C under a pressure of 0.1 to 5mmHg so as to generate a cyclic oligomer.

9. The method for producing a cyclic lactic acid oligomer according to any one of claims 1 to 8, wherein cyclic lactic acid oligomers are selectively produced while substantially no chain lactic acid oligomers are produced.

10. The method for producing a cyclic lactic acid oligomer according to any one of claims 1 to 9, wherein the ratio of cyclic lactic acid oligomers to total lactic acid oligomers in the reaction product is 80% by weight or more.

11. A cyclic lactic acid oligomer produced by the method for producing a cyclic lactic acid oligomer according to any one of claims 1 to 10.

12. The cyclic lactic acid oligomer according to claim 11, which is substantially free of chain lactic acid oligomers.
